# EUROPEAN PATENT APPLICATION

(11) **EP 4 588 439 A1**
(43) Date of publication of application: **23.07.2025**
(21) Application number: 23865496.6
(22) Date of filing: 11.09.2023
(51) Int. Cl.: A61B 5/274, A61B 5/268

(54) **ELECTRODE MEMBER AND BIOELECTRIC SIGNAL MEASURING ELECTRODE**

(30) Priority: 15.09.2022 JP 2022147187
(71) Applicant: NOK Corporation, Tokyo 105-8585 (JP)
(72) Inventor: HAYASHI, Taisei, Fujisawa-shi, Kanagawa 251-0042 (JP); KUBO, Masayuki, Fujisawa-shi, Kanagawa 251-0042 (JP); HAYASHI, Takahiro, Fujisawa-shi, Kanagawa 251-0042 (JP)
(74) Representative: Global IP Europe Patentanwaltskanzlei
(86) International application number: PCT/JP2023/033093
(87) International publication number: WO 2024/058143

(57) **Abstract**

An electrode member (3) for a bioelectric signal measuring electrode is made of an electroconductive material, electrically connected to a wire (6a), and coupled to a metal member (4) including a hole (4a). The electrode member (3) includes a plate portion (3a) to be set in contact with a living body (2), a head portion (3b) to be fitted into the hole (4a) of the metal member (4), and a neck portion (3c). The head portion (3b) includes a base end portion (3d), a distal end portion (3e) opposite to the base end portion (3d), a peripheral surface portion (3f) between the base end portion (3d) and the distal end portion (3e), and a slit (3g) formed on the distal end portion (3e) and the peripheral surface portion (3f). The head portion (3b) has a shape tapered toward the distal end portion (3e). The base end portion (3d) is fixed to the neck portion (3c). The neck portion (3c) connects the plate portion (3a) to the head portion (3b).

## Description

### FIELD

The present disclosure relates to an electrode member and a bioelectric signal measuring electrode.

### BACKGROUND

Known electrodes are set in contact with a living body in order to measure bioelectric signals. Such an electrode is placed on a head, a chest, an abdomen, or the like of a living body such as a human body.

Japanese Patent Application Laid-Open Publication No. 2021-159216 (hereinafter, referred to as Patent Literature 1) discloses a bioelectric signal measuring electrode that includes an electrode member and a metal member. The electrode member is made of electroconductive rubber. The electrode member is set in contact with a living body. The metal member resembles a snap button in such a way that the electrode member is fitted into the metal member. The electrode made of the electroconductive rubber is softer than a metal electrode, and thus can secure a larger area contacting with a living body without causing painful pressure. According to the bioelectric signal measuring electrode disclosed in Patent Literature 1, the electrode member made of the electroconductive rubber is not easily detached from the metal member after being once fitted into the metal member.

### BRIEF SUMMARY

### TECHNICAL PROBLEM

It is desirable that the electrode member made of the electroconductive rubber and used in combination with the metal member is easily fitted into the metal member.

In view of it, an object of the present disclosure is to provide an electrode member that is easily fitted into a metal member and is not easily detached from the metal member after being once fitted into the metal member, and to provide a bioelectric signal measuring electrode that includes the metal member and the electrode member.

### SOLUTION TO PROBLEM

A first aspect of the present disclosure provides an electrode member for a bioelectric signal measuring electrode, the electrode member being made of an electroconductive material, electrically connected to a wire, and coupled to a metal member including a hole, the electrode member including:
a plate portion to be set in contact with a living body;
a head portion to be fitted into the hole of the metal member, the head portion including
   a base end portion,
   a distal end portion opposite to the base end portion,
   a peripheral surface portion between the base end portion and the distal end portion, and
   a slit formed on the distal end portion and the peripheral surface portion, wherein
   the head portion has a shape tapered toward the distal end portion; and
a neck portion to which the base end portion is fixed, the neck portion connecting the plate portion to the head portion.

A second aspect of the present disclosure provides a bioelectric signal measuring electrode including:
a metal member including a hole; and
the electrode member.

### ADVANTAGEOUS EFFECTS

The present disclosure can provide an electrode member that is easily fitted into a metal member and is not easily detached from the metal member after being once fitted into the metal member, and can provide a bioelectric signal measuring electrode that includes the metal member and the electrode member.

### BRIEF DESCRIPTION OF DRAWINGS

FIG. 1 is a sectional view of a bioelectric signal measuring electrode according to a first embodiment.
FIG. 2 is a front view of an electrode member of the bioelectric signal measuring electrode in FIG. 1.
FIG. 3 is a plan view of a head portion and a neck portion of the electrode member in FIG. 2.
FIG. 4 is a cross-sectional view taken along the IV-IV line in FIG. 2.
FIG. 5 is a perspective view of the head portion and the neck portion of the electrode member in FIG. 2 that are viewed from obliquely below.
FIG. 6 is a plan view of a head portion and a neck portion of an electrode member according to a modified example of the first embodiment.
FIG. 7 is a plan view of a head portion and a neck portion of an electrode member according to another modified example of the first embodiment.
FIG. 8 is a perspective view of a metal member of the bioelectric signal measuring electrode in FIG. 1.
FIG. 9 is a sectional view taken along the IX-IX line in FIG. 8.
FIG. 10 is a sectional view illustrating an assembling step of the bioelectric signal measuring electrode in FIG. 1.
FIG. 11 is a front view of an electrode member according to another modified example of the first embodiment.
FIG. 12 is a front view of an electrode member according to another modified example of the first embodiment.
FIG. 13 is a sectional view of a bioelectric signal measuring electrode according to a second embodiment.
FIG. 14 is a front view of an electrode member of the bioelectric signal measuring electrode in FIG. 13.
FIG. 15 is a plan view of a head portion and a neck portion of the electrode member in FIG. 14.
FIG. 16 is a perspective view of the head portion and the neck portion of the electrode member in FIG. 14 that are viewed from obliquely below.
FIG. 17 is a perspective view of a metal member of the bioelectric signal measuring electrode in FIG. 13.
FIG. 18 is a sectional view taken along the XVIII-XVIII line in FIG. 17.
FIG. 19 is a sectional view illustrating an assembling step of the bioelectric signal measuring electrode in FIG. 13.
FIG. 20 is a front view of an electrode member according to a modified example of the second embodiment.
FIG. 21 is a front view of an electrode member according to another modified example of the second embodiment.

### DETAILED DESCRIPTION

The following describes various embodiments of the present disclosure with reference to the accompanying drawings. Reduction scales of the drawings are not necessarily precise, and some features are emphasized or omitted in the drawings in some cases.

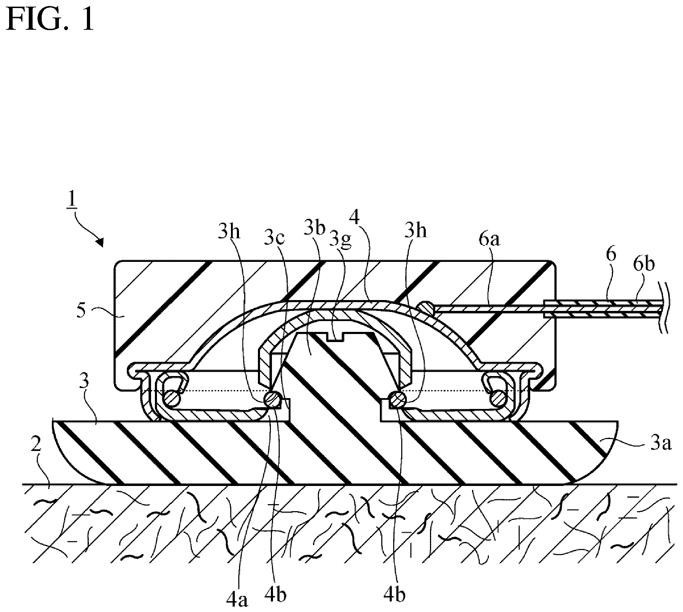

As illustrated in FIG. 1, a bioelectric signal measuring electrode 1 according to a first embodiment includes an electrode member 3 made of electroconductive rubber, a metal member 4, a protection cover 5 made of resin, and a cable 6.

The cable 6 includes a wire 6a and an insulator 6b. The insulator 6b covers the wire 6a. The wire 6a includes an end portion exposed from the insulator 6b and electrically connected to the metal member 4.

The protection cover 5 is positioned on an upper side of the metal member 4. The protection cover 5 covers an upper portion of the metal member 4, and surrounds the end portion of the wire 6a. The protection cover 5 is fixed to the metal member 4 and the cable 6.

The metal member 4 is a female snap button (snap fastener) itself including a hole 4a, or is a member having a shape similar to that of a female snap button.

The electrode member 3 is made of the electroconductive rubber. The electroconductive rubber may be silicone rubber in which electric-conductor particles (e.g., electroconductive carbon particles or silver particles) are dispersed. The electrode member 3 may include layers (e.g., a layer of silicone rubber in which electroconductive carbon particles are dispersed and a layer of silicone rubber in which a silver powder is dispersed) formed of two types of electroconductive rubber.

The electrode member 3 made of the electroconductive rubber is coupled to the metal member 4. The electrode member 3 is set in contact with a living body 2 such as a human body. An electric signal from the living body 2 passes through the electrode member 3 and the metal member 4 to be transmitted to the wire 6a. An end portion included in the cable 6 and opposite to the metal member 4 is connected to an electrical measuring device (not illustrated in the drawings) such as an ammeter. A plurality of the cables 6 connected to a plurality of the respective bioelectric signal measuring electrodes 1 are connected to the electrical measuring devices. A plurality of the bioelectric signal measuring electrodes 1 are placed at various parts of the living body such as a human body.

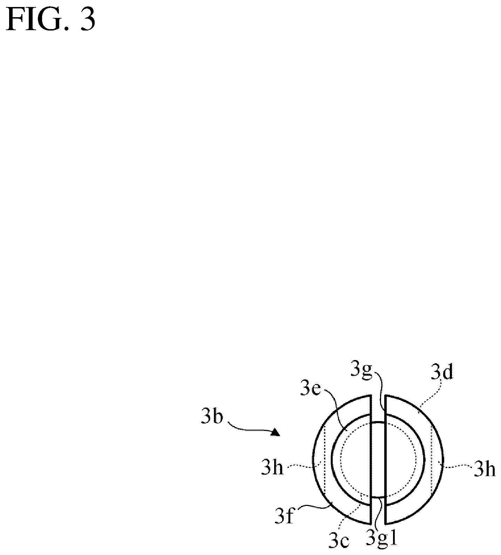

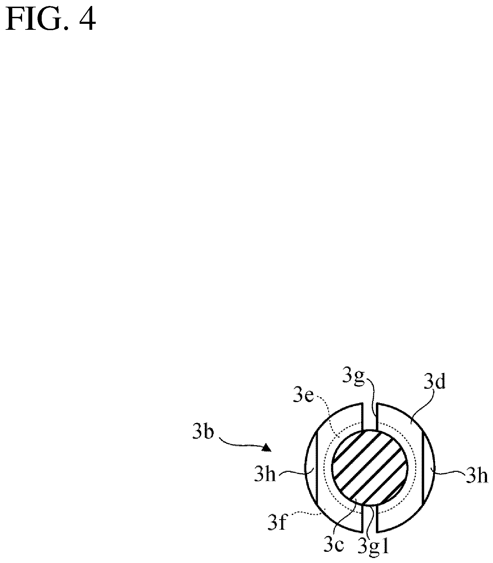

As illustrated in FIG. 1 to FIG. 5, the electrode member 3 includes a plate portion 3a, a head portion 3b, and a neck portion 3c.

The plate portion 3a has a disk shape for example, but may have a different shape. The plate portion 3a includes a lower surface to be set in contact with the living body 2. The head portion 3b is fitted into the hole 4a of the metal member 4. The neck portion 3c connects the plate portion 3a to the head portion 3b.

The head portion 3b includes a base end portion 3d, a distal end portion 3e, and a peripheral surface portion 3f. The base end portion 3d is fixed to the neck portion 3c. The base end portion 3d in the present embodiment is a flat surface parallel to an upper surface of the plate portion 3a. The distal end portion 3e is positioned on an opposite side of the base end portion 3d. The distal end portion 3e in the present embodiment is a flat surface parallel to the upper surface of the plate portion 3a. The peripheral surface portion 3f is positioned between the base end portion 3d and the distal end portion 3e.

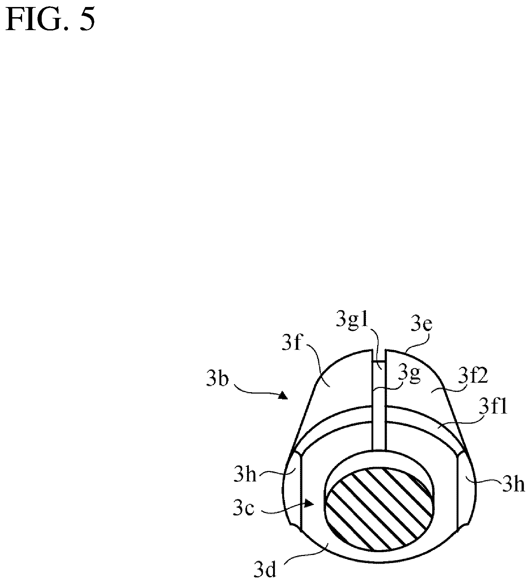

The head portion 3b has a shape tapered toward the distal end portion 3e. As illustrated in FIG. 5, the peripheral surface portion 3f in the present embodiment includes a lower peripheral surface portion 3f1 having a substantially cylindrical shape and an upper peripheral surface portion 3f2 having a substantially truncated-cone shape. The upper peripheral surface portion 3f2 is coaxial with the lower peripheral surface portion 3f1.

The neck portion 3c in the present embodiment has a cylindrical shape coaxial with the head portion 3b. The neck portion 3c has a diameter smaller than a diameter of the lower peripheral surface portion 3f1. Thus, a maximum-dimension part of the head portion 3b has a cross-sectional area larger than a cross-sectional area of the neck portion 3c.

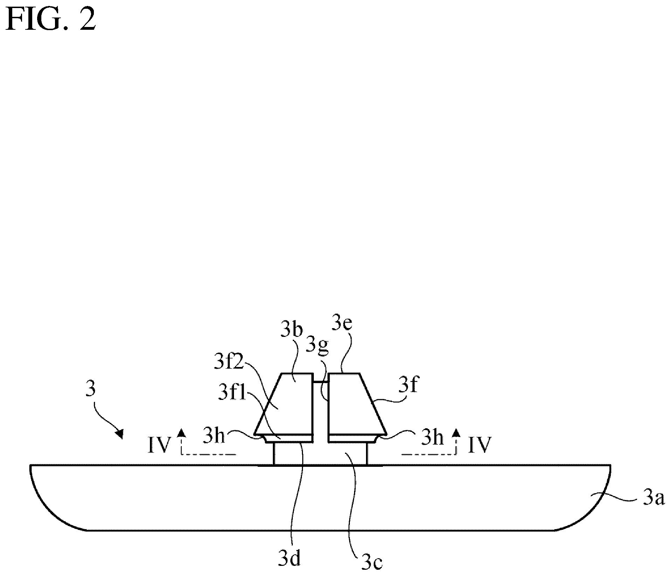

The base end portion 3d includes the surface that faces toward a side of the neck portion 3c and on which a pair of grooves 3h are formed in parallel to each other. As illustrated in FIG. 2 and FIG. 5, the grooves 3h each have an arc-shaped contour. The grooves 3h in the present embodiment are formed also in the lower peripheral surface portion 3f1. When the grooves 3h are not formed, the lower peripheral surface portion 3f1 has a cylindrical shape. However, because of the grooves 3h, an lower end of the lower peripheral surface portion 3f1 has a shape defined by two arcs and two line segments.

A slit 3g is formed on the distal end portion 3e and the peripheral surface portion 3f of the head portion 3b. The slit 3g is formed on one straight line passing through the central axis of the head portion 3b. The slit 3g equally divides the distal end portion 3e into two parts, and equally divides the peripheral surface portion 3f into two parts. In the present embodiment, a bottom surface 3g1 of the slit 3g formed on the peripheral surface portion 3f is flush with an outer peripheral surface of the neck portion 3c. However, the bottom surface 3g1 is not limited to this.

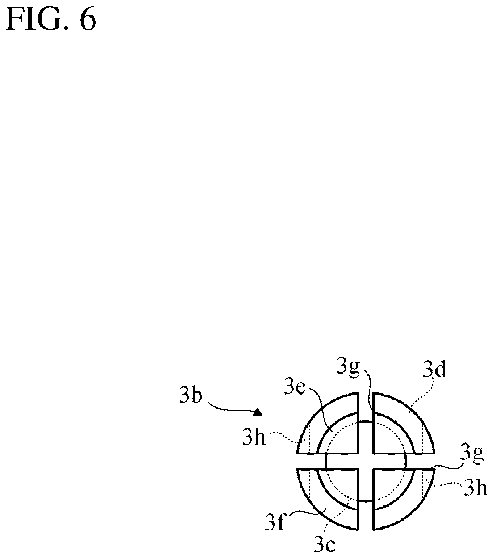

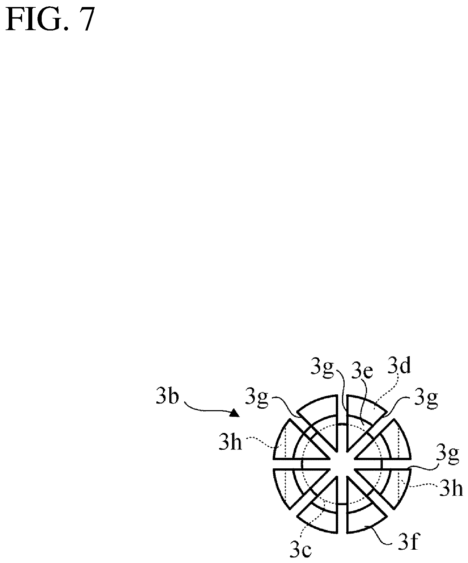

The head portion 3b in the present embodiment includes the one slit 3g. However, the head portion 3b is not limited to this. As illustrated in FIG. 6, the head portion 3b may include two slits 3g. The two slits 3g are formed on the distal end portion 3e and the peripheral surface portion 3f. Thereby, the distal end portion 3e may be equally divided into four parts, and the peripheral surface portion 3f may be equally divided into four parts. As illustrated in FIG. 7, the head portion 3b may include four slits 3g. The four slits 3g are formed on the distal end portion 3e and the peripheral surface portion 3f. Thereby, the distal end portion 3e may be equally divided into eight parts, and the peripheral surface portion 3f may be equally divided into eight parts.

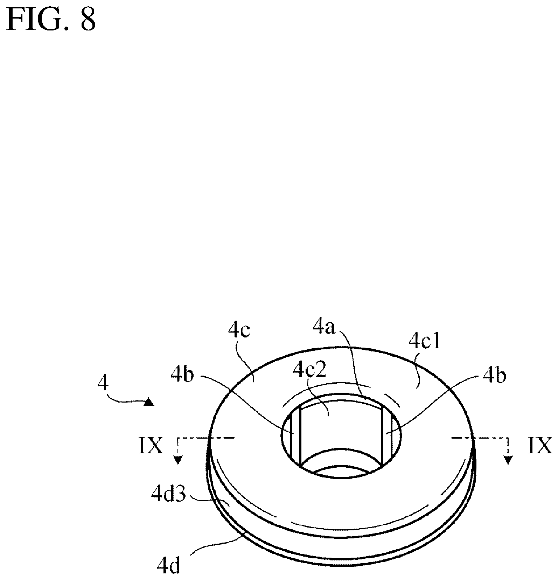

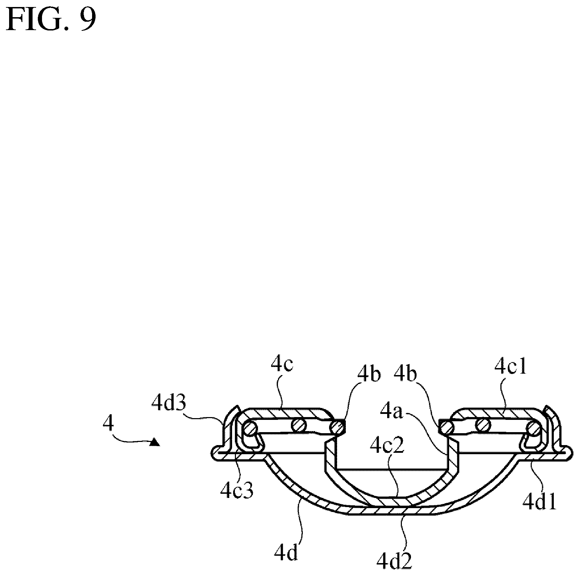

As illustrated in FIG. 8 and FIG. 9, the metal member 4 includes an inner ring 4c, an outer ring 4d, and a pair of spring bar members 4b.

The inner ring 4c includes a flat annular portion 4c1, a dome portion 4c2, and an outer edge portion 4c3. The dome portion 4c2 is formed at a center of the flat annular portion 4c1. An intemal cavity of the dome portion 4c2 serves as the hole 4a into which the head portion 3b of the electrode member 3 is inserted. The outer edge portion 4c3 is formed at an outer edge of the flat annular portion 4c1. The flat annular portion 4c1, the dome portion 4c2, and the outer edge portion 4c3 are formed by one metal plate.

A pair of the spring bar members 4b are attached to the inner ring 4c. A part of each of the spring bar members 4b is positioned in the hole 4a. A pair of the spring bar members 4b include the parts that are positioned in the hole 4a and that are parallel to each other.

The outer ring 4d includes a flat annular portion 4d1, a dome portion 4d2, and an outer edge portion 4d3. The flat annular portion 4d1 is positioned in parallel to the flat annular portion 4c1 of the inner ring 4c. The dome portion 4d2 is formed at a center of the flat annular portion 4c1. The dome portion 4d2 surrounds the dome portion 4c2 of the metal member 4. The outer edge portion 4d3 is formed at an outer edge of the flat annular portion 4d1. The outer edge portion 4d3 surrounds the outer edge portion 4c3 of the inner ring 4c. The outer edge portion 4d3 and the outer edge portion 4c3 are fixed to each other by caulking.

The metal member 4 includes a pair of the spring bar members 4b. The part of each of the spring bar members 4b is positioned in the hole 4a. The parts included in the spring bar members 4b and positioned in the hole 4a are parallel to each other.

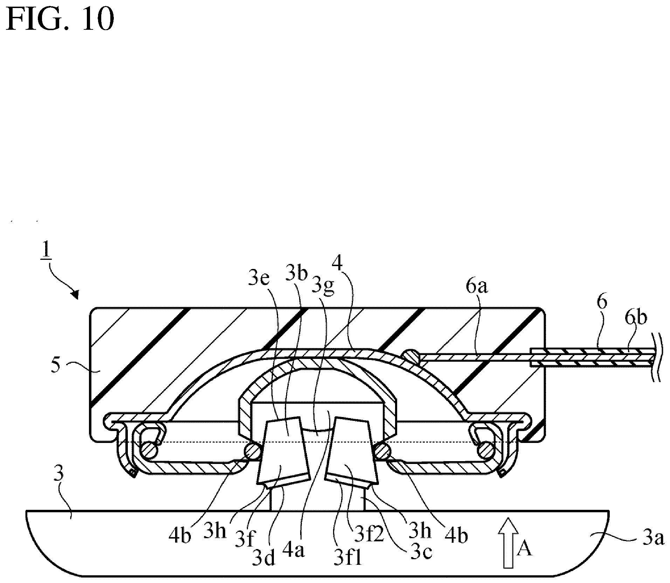

FIG. 10 is a sectional view illustrating an assembling step of the bioelectric signal measuring electrode 1. However, FIG. 10 depicts the electrode member 3 by a front view instead of by a sectional view.

Assembling the bioelectric signal measuring electrode 1 includes making the electrode member 3 approach the metal member 4 as indicated by the arrow A, and inserting the head portion 3b of the electrode member 3 into the hole 4a of the metal member 4. In other words, the head portion 3b of the electrode member 3 is fitted into the hole 4a of the metal member 4. At this time, the head portion 3b having the tapered shape is gradually inserted into the hole 4a.

The slit 3g is formed on the distal end portion 3e and the peripheral surface portion 3f of the head portion 3b. Thus, the head portion 3b is easily elastically-shrinkable in a radial direction. When the head portion 3b is fitted into the hole 4a, the head portion 3b is shrunken in the radial direction so that the head portion 3b is easily fitted into the hole 4a of the metal member 4. Even when a material of the head portion 3b has low elasticity, the head portion 3b can be easily fitted into the hole 4a.

The base end portion 3d of the head portion 3b has a cross-sectional area larger than a cross-sectional area of the neck portion 3c. Thus, after the head portion 3b is once fitted into the hole 4a of the metal member 4 as illustrated in FIG. 1, the head portion 3b is not easily detached from the metal member 4.

The base end portion 3d in the present embodiment includes the surface that faces toward a side of the neck portion 3c and on which a pair of the grooves 3h are formed in parallel to each other. The metal member 4 includes a pair of the spring bar members 4b. The spring bar members 4b each include the part positioned in the hole 4a. The parts included in the spring bar members 4b and positioned in the hole 4a are parallel to each other. When the head portion 3b is fitted into the hole 4a, the spring bar members 4b are fitted into the grooves 3h. For this reason, after the head portion 3b is once fitted into the hole 4a of the metal member 4, the head portion 3b is not easily detached from the metal member 4. Further, the respective spring bar members 4b fitted into the grooves 3h prevent the head portion 3b from rotating relative to the metal member 4. In other words, a posture of the electrode member 3 can be kept constant relative to the metal member 4.

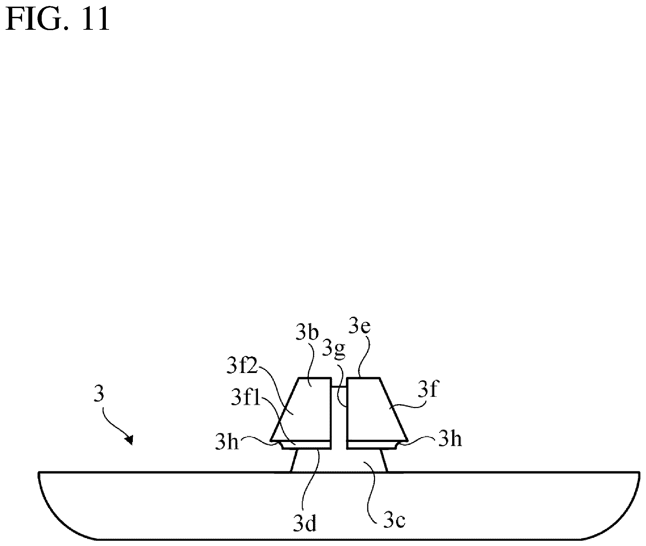

The neck portion 3c in the present embodiment has the cylindrical shape. However, the neck portion 3c may have a truncated-cone shape, as illustrated in FIG. 11.

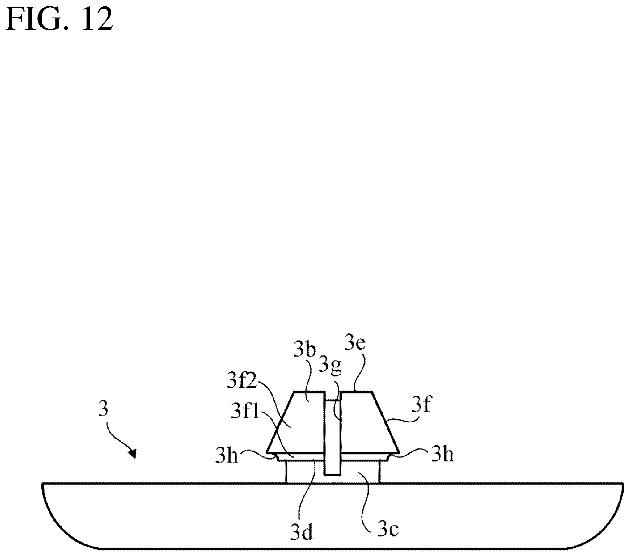

In the present embodiment, the slit 3g is formed on the head portion 3b. However, the slit 3g may further extend to be formed also in the neck portion 3c, as illustrated in FIG. 12.

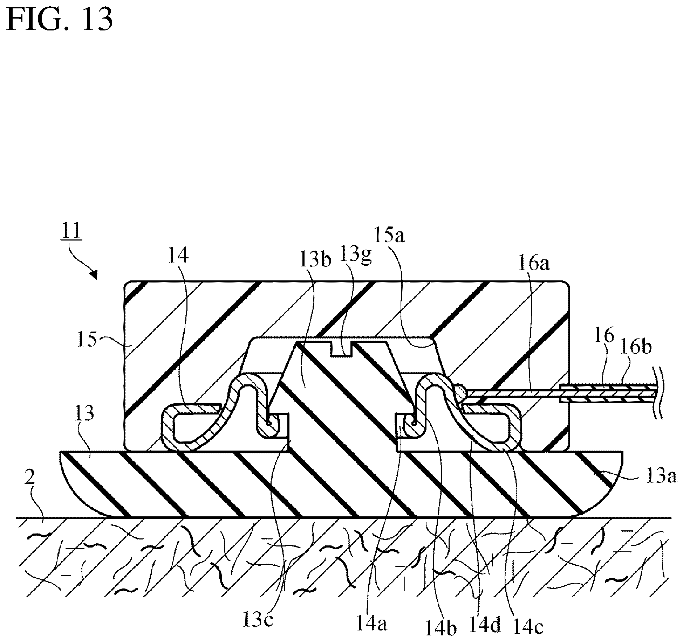

As illustrated in FIG. 13, a bioelectric signal measuring electrode 11 according to a second embodiment includes an electrode member 13 made of electroconductive rubber, a metal member 14, a protection cover 15 made of resin, and a cable 16.

The cable 16 includes a wire 16a and an insulator 16b. The insulator 16b covers the wire 16a. The wire 16a includes an end portion exposed from the insulator 16b and electrically connected to the metal member 14.

The protection cover 15 is positioned on an upper side of the metal member 14. The protection cover 15 covers an upper portion of the metal member 14, and surrounds the end portion of the wire 16a. The protection cover 15 is fixed to the metal member 14 and the cable 16.

The metal member 14 is a female snap button (snap fastener) itself including a hole 14a, or is a member having a shape similar to that of a female snap button.

The electrode member 13 in the present embodiment is made of the electroconductive rubber as in the case of the electrode member 3 in the first embodiment.

The electrode member 13 made of the electroconductive rubber is coupled to the metal member 14. The electrode member 13 is set in contact with a living body 2 such as a human body. An electric signal from the living body 2 passes through the electrode member 13 and the metal member 14 to be transmitted to the wire 16a. An end portion included in the cable 16 and opposite to the metal member 14 is connected to an electrical measuring device (not illustrated in the drawings) such as an ammeter. A plurality of the cables 16 connected to a plurality of the respective bioelectric signal measuring electrodes 11 are connected to the electrical measuring devices. A plurality of the bioelectric signal measuring electrodes 11 are placed at various parts of the living body such as a human body.

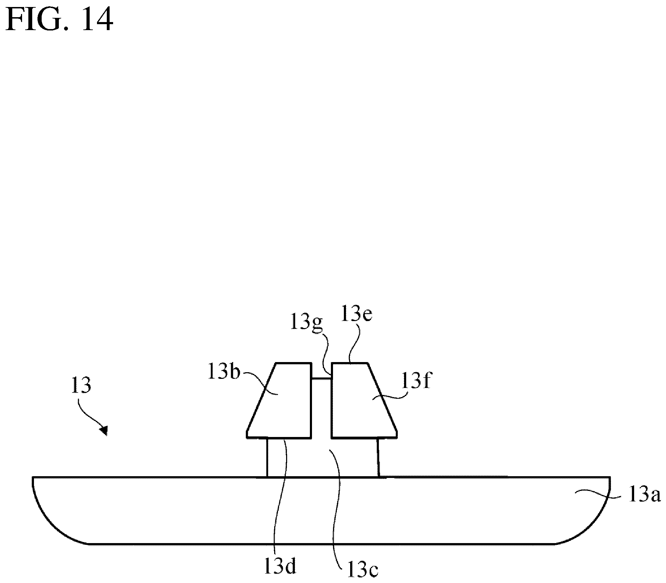

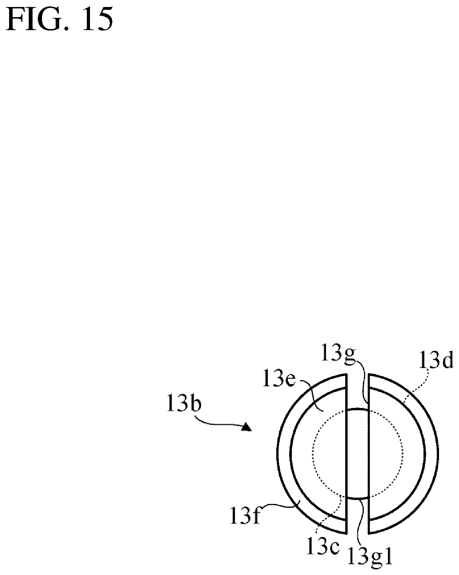

As illustrated in FIG. 13 to FIG. 16, the electrode member 13 includes a plate portion 13a, a head portion 13b, and a neck portion 13c.

The plate portion 13a has a disk shape for example, but may have a different shape. The plate portion 13a includes a lower surface to be set in contact with the living body 2. The head portion 13b is fitted into the hole 14a of the metal member 14. The neck portion 13c connects the plate portion 13a to the head portion 13b.

The head portion 13b includes a base end portion 13d, a distal end portion 13e, and a peripheral surface portion 13f. The base end portion 13d is fixed to the neck portion 13c. The base end portion 13d in the present embodiment is a flat surface parallel to an upper surface of the plate portion 13a. The distal end portion 13e is positioned on an opposite side of the base end portion 13d. The distal end portion 13e in the present embodiment is a flat surface parallel to the upper surface of the plate portion 13a. The peripheral surface portion 13f is positioned between the base end portion 13d and the distal end portion 13e.

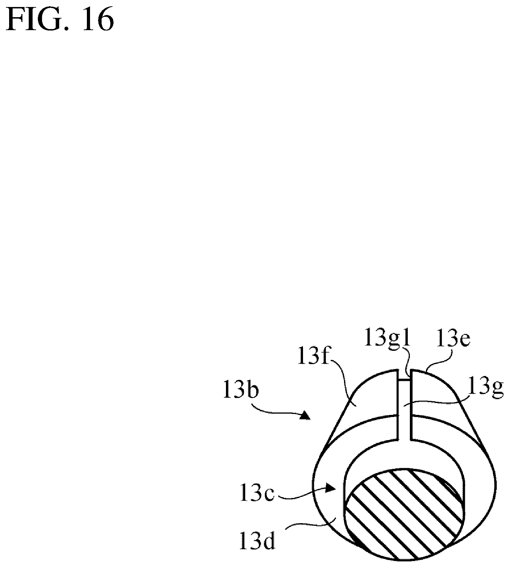

The head portion 13b has a shape tapered toward the distal end portion 13e. As illustrated in FIG. 16, the head portion 13b in the present embodiment has a substantially truncated-cone shape.

The neck portion 13c in the present embodiment has a cylindrical shape coaxial with the head portion 13b. The neck portion 13c has a diameter smaller than the maximum diameter of the head portion 13b. Thus, a maximum-dimension part of the head portion 13b has a cross-sectional area larger than a cross-sectional area of the neck portion 13c.

A slit 13g is formed on the distal end portion 13e and the peripheral surface portion 13f of the head portion 13b. The slit 13g is formed on a straight line passing through the central axis of the head portion 13b. The slit 13g equally divides the distal end portion 13e into two parts, and equally divides the peripheral surface portion 13f into two parts. In the present embodiment, a bottom surface 13g1 of the slit 13g formed on the peripheral surface portion 13f is flush with an outer peripheral surface of the neck portion 13c. However, the bottom surface 13g1 is not limited to this.

The head portion 13b in the present embodiment includes the one slit 13g. However, the number of the slits 13g is not limited to that in the present embodiment, as described above with reference to FIG. 6 and FIG. 7 concerning the first embodiment.

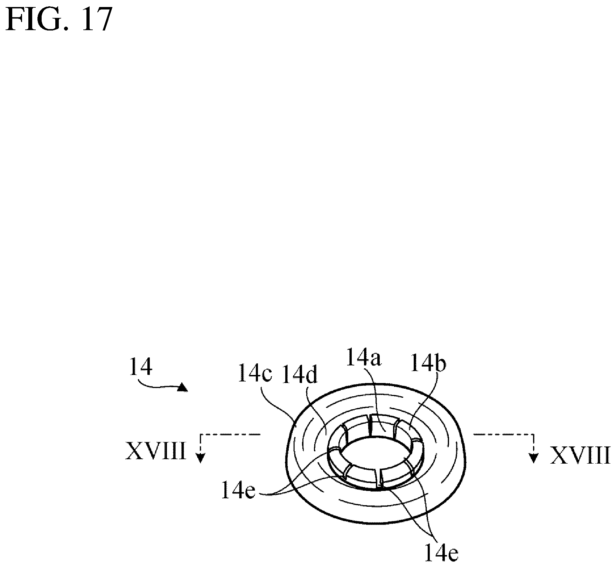

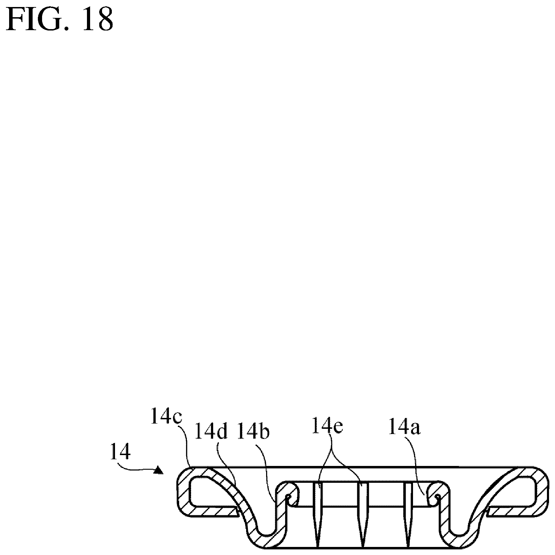

As illustrated in FIG. 17 and FIG. 18, the metal member 14 includes a cylindrical portion 14b, an outer edge portion 14c, and a connection curved portion 14d. The cylindrical portion 14b is positioned at a center of the metal member 14. An internal cavity of the cylindrical portion 14b serves as the hole 14a into which the head portion 13b of the electrode member 13 is inserted. The outer edge portion 14c is positioned at an outer edge of the metal member 14 coaxially with the cylindrical portion 14b. The connection curved portion 14d connects the cylindrical portion 14b to the outer edge portion 14c. The cylindrical portion 14b, the outer edge portion 14c, and the connection curved portion 14d are formed by one metal plate.

A plurality of slits 14e are formed on the cylindrical portion 14b at an equal angle interval. Each of the slits 14e extends along an axial direction of the metal member 14.

As illustrated in FIG. 13, a hole 15a is formed in the protection cover 15. The hole 15a communicates with the hole 14a of the metal member 14. A part of the head portion 13b of the electrode member 13 is positioned in the hole 15a.

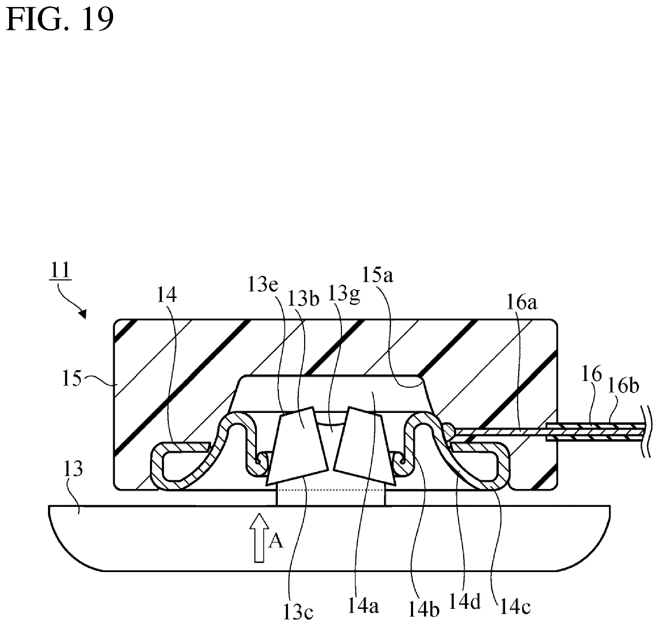

FIG. 19 is a sectional view illustrating an assembling step of the bioelectric signal measuring electrode 11. However, FIG. 19 depicts the electrode member 13 by a front view instead of by a sectional view.

Assembling the bioelectric signal measuring electrode 11 includes making the electrode member 13 approach the metal member 14 as indicated by the arrow A, and inserting the head portion 13b of the electrode member 13 into thehole 14a of the metal member 14. In other words, the head portion 13b of the electrode member 13 is fitted into the hole 14a of the metal member 14. At this time, the head portion 13b having the tapered shape is gradually inserted into the hole 14a.

The slit 13g is formed on the distal end portion 13e and the peripheral surface portion 13f of the head portion 13b. Thus, the head portion 13b is easily elastically-shrinkable in a radial direction. When the head portion 13b is fitted into the hole 14a, the head portion 13b is shrunken in the radial direction so that the head portion 13b is easily fitted into the hole 14a of the metal member 14. Even when a material of the head portion 13b has low elasticity, the head portion 13b can be easily fitted into the hole 14a.

The base end portion 13d of the head portion 13b has a cross-sectional area larger than a cross-sectional area of the neck portion 13c. Thus, after the head portion 13b is once fitted into the hole 14a of the metal member 14 as illustrated in FIG. 13, the head portion 13b is not easily detached from the metal member 14.

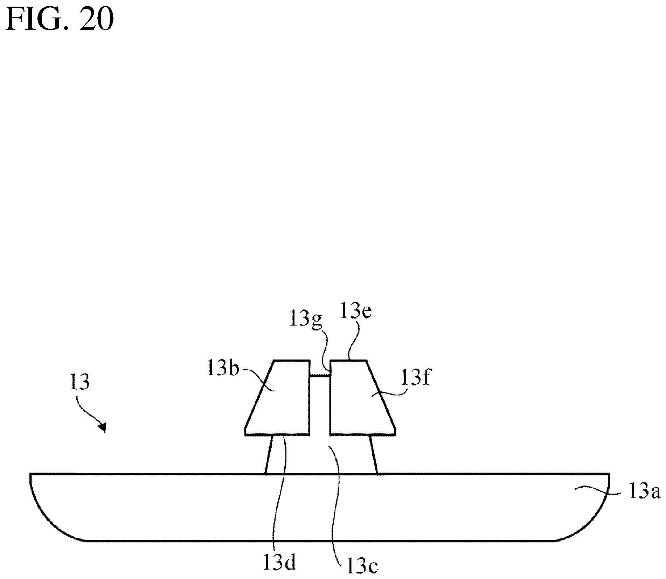

The neck portion 13c in the present embodiment has the cylindrical shape. However, the neck portion 13c may have a truncated-cone shape, as illustrated in FIG. 20.

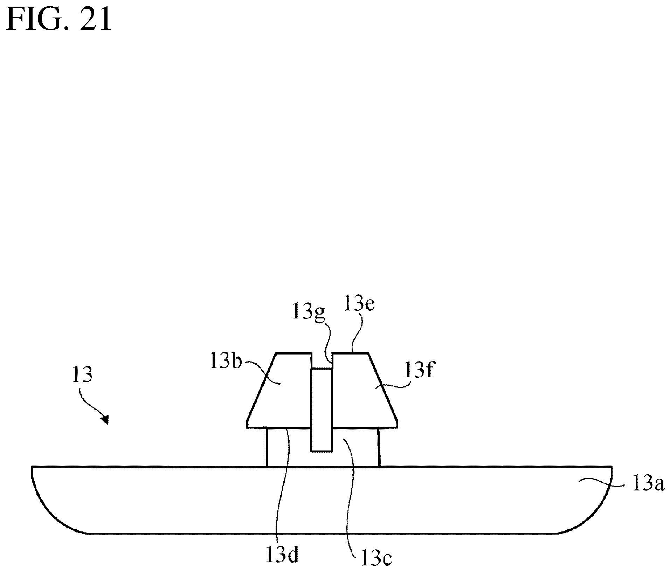

In the present embodiment, the slit 13g is formed on the head portion 13b. However, the slit 13g may further extend to be formed also in the neck portion 13c, as illustrated in FIG. 21.

The present disclosure is illustrated in the drawings and described above with reference to the preferred embodiments of the present disclosure. However, it would be understood for those skilled in the art that forms and details can be modified without departing from the scope of the invention described in claims. Such modifications, improvements, and alterations are encompassed within the scope of the present disclosure.

### Reference Signs List

1, 11 bioelectric signal measuring electrode
2 living body
3, 13 electrode member
3a, 13a plate portion
3b, 13b head portion
3c, 13c neck portion
3d, 13d base end portion
3e, 13e distal end portion
3f, 13f peripheral surface portion
3g, 13g slit
3h groove
4, 14 metal member
4a, 14a hole
4b spring bar member
6a, 16a wire

## Claims

1. An electrode member for a bioelectric signal measuring electrode, the electrode member being made of an electroconductive material, electrically connected to a wire, and coupled to a metal member including a hole, the electrode member comprising:
a plate portion to be set in contact with a living body;
a head portion to be fitted into the hole of the metal member, the head portion including
a base end portion,
a distal end portion opposite to the base end portion,
a peripheral surface portion between the base end portion and the distal end portion, and
a slit formed on the distal end portion and the peripheral surface portion, wherein
the head portion has a shape tapered toward the distal end portion; and
a neck portion to which the base end portion is fixed, the neck portion connecting the plate portion to the head portion.

2. The electrode member according to claim 1, wherein
the base end portion includes a surface that faces toward a side of the neck portion and on which a pair of grooves are formed in parallel to each other.

3. A bioelectric signal measuring electrode comprising:
the metal member including the hole; and
the electrode member according to claim 1 or 2.

4. The bioelectric signal measuring electrode according to claim 3, wherein
the metal member includes a pair of spring bar members,
the spring bar members each include a part positioned in the hole,
the parts included in the spring bar members and positioned in the hole are parallel to each other, and
the spring bar members are fitted into the respective grooves.
